# EUROPÄISCHE PATENTANMELDUNG

(11) **EP 3 851 082 A1**
(43) Veröffentlichungstag der Anmeldung: **21.07.2021**
(21) Anmeldenummer: 20152365.1
(22) Anmeldetag: 17.01.2020
(51) Int. Cl.: A61F 5/08

(54) **VORRICHTUNG ZUR VERBESSERUNG DER ATMUNG**

(71) Anmelder: Herbst, Martin, 5090 Lofer (AT)
(72) Erfinder: Herbst, Martin, 5090 Lofer (AT)
(74) Vertreter: Grünecker Patent- und Rechtsanwälte PartG mbB

(57) **Zusammenfassung**

Die Erfindung betrifft eine Vorrichtung zur Verbesserung der Atmung, umfassend mindestens einen Naseneinsatz, der dazu vorgesehen ist, in ein Nasenloch eines Benutzers eingeführt zu werden, wobei der mindestens eine Naseneinsatz einen konusförmigen Grundkörper, ein Durchgangsloch, das sich entlang der Hauptachse des Grundkörpers erstreckt, und ein spiralförmiges Element, das an der Innenseite des Grundkörpers angeordnet ist und sich von der Basis des Grundkörpers bis zur Spitze des Grundkörpers erstreckt.

## Beschreibung

### Technisches Gebiet

Die Erfindung betrifft eine Vorrichtung zur Verbesserung der Atmung eines Benutzers. Die Erfindung betrifft weiterhin die Verwendung einer solchen Vorrichtung zur Verbesserung der Atmung.

### Hintergrund

Die Nase gehört anatomisch zu den äußeren und oberen Atemwegen und ist ein wichtiger Teil des menschlichen Atemtrakts. Sie beherbergt die Nasenlöcher und die Nasenhöhle, wobei die Nasenlöcher in das Innere der Nase führen, jeweils zuerst in den Nasenvorhof, dann in die eigentliche Nasenhöhle. Die Nasenhöhle ist durch die Nasenscheidewand in zwei getrennte Abteilungen gegliedert und von einer Schleimhaut ausgekleidet. Die linke und rechte Nasenhöhle sind jeweils durch die knöchern gestützten Nasenmuscheln untergliedert, die drei Nasengänge abgrenzen. Der obere Nasengang, auch Riechgang genannt, beherbergt in seinem hinteren Teil das Geruchsorgan, und der mittlere Nasengang mündet in die Nasennebenhöhlen. Der untere Nasengang ist der größte der drei Nasengänge.

Obwohl alle Nasengänge über die Choanenöffnung in den Nasenrachen führen und damit als Luftwege dienen, wird die meiste Atemluft über den unteren Nasengang transportiert, da er aufgrund seiner Größe und Lage den direktesten Weg von der Nasenöffnung zum Nasenrachen darstellt. Daher wird der untere Nasengang auch als Atemgang bezeichnet.

Eine Verengung oder sonstige anatomische Änderung der Nasenhöhle oder der Nasengänge führt oft zu einer Beeinträchtigung der Atmung durch die Nase. Eine solche Verengung kann vielfältige Gründe haben. So kann sie z.B. aufgrund eines Anschwellens der Schleimhäute der Nasenhöhle bei einer Rhinitis oder Erkältung auftreten. Beispielsweise kann aber auch eine Nasenscheidewandverkrümmung zu asymmetrischen Strömungsverhältnissen in beiden Nasenhälften führen. Solche Veränderungen können beispielsweise die Erholung während des Schlafs beeinträchtigen, da nicht ausreichend Luft durch die Nase aufgenommen werden kann. Sie können sich zudem auch in Schnarchen während des Schlafs äußern.

Vorrichtungen zur Verbesserung der Atmung durch die Nase sind grundsätzlich bekannt. So offenbart beispielsweise die EP 3 498 237 A1 eine schlauchförmige Vorrichtung, die in einen Nasengang eingeführt wird und diesem dadurch eine gewünschte Form geben kann. Dadurch soll ein verbesserter Luftdurchfluss durch die Nase erreicht werden. Diese bekannte Vorrichtung wird individuell für Patienten angepasst, um den Nasengang optimal zu formen. Allerdings wird diese schlauchförmige Vorrichtung nahezu vollständig in den Nasengang eingeführt. Daher gestaltet sich ihre Verwendung, insbesondere das Entfernen, aufwändig. Weiterhin kann die bekannte Vorrichtung zwar eine eventuell vorliegende Verengung des Nasenkanals ausgleichen, sie erlaubt aber keine Verbesserung der Atmung, falls der Nasenkanal nicht verengt oder blockiert ist. Weiterhin muss diese Vorrichtung, wie oben erwähnt, für jeden Patienten individuell angepasst werden, was eine Massenfertigung schwierig macht.

Die Aufgabe der vorliegenden Erfindung ist es daher, eine Vorrichtung zur Verbesserung der Atmung durch die Nase bereitzustellen, die einfach verwendet werden kann.

### Zusammenfassung

Diese Aufgabe wird gelöst durch eine Vorrichtung gemäß Anspruch 1. Bevorzugte Ausführungsformen sind in den abhängigen Ansprüchen beschrieben.

Die erfindungsgemäße Vorrichtung umfasst mindestens einen Naseneinsatz, der dazu vorgesehen ist, in ein Nasenloch eines Benutzers eingeführt zu werden. Dabei umfasst der mindestens eine Naseneinsatz einen konusförmigen Grundkörper, sowie ein Durchgangsloch, das sich entlang der Hauptachse des Grundkörpers erstreckt. Weiterhin umfasst der Naseneinsatz ein spiralförmiges Element, das innerhalb des Grundkörpers angeordnet ist und sich von der Basis des Grundkörpers bis zur Spitze des Grundkörpers erstreckt. Mit anderen Worten verläuft das spiralförmige Element im Inneren des Grundkörpers in Form einer Schraublinie von der Basis des Grundkörpers bis zu seiner Spitze. Insbesondere kann die Außenseite des Grundkörpers dazu vorgesehen sein, bei Verwendung der Vorrichtung in direktem Kontakt mit dem Nasenvorhof und/oder der Nasenhöhle zu stehen

Der Grundkörper ist konusförmig, worunter hier eine Form verstanden wird, die sich nach oben hin verjüngt, um sich dem Inneren der Nase anzupassen. Insbesondere kann es sich dabei um einen Kegel- oder Pyramidenstumpf handeln. Es kann sich um einen geraden oder einen schiefen Kegel- oder Pyramidenstumpf handeln. Die Begriffe "auptachse", "Basis", und "Mantel" bezeichnen in der üblichen Weise die entsprechenden Elemente eines Kegels bzw. einer Pyramide. Mit der "Spitze" wird der verjüngte Teil des Grundkörpers bezeichnet.

Wenn im Folgenden die Begriffe "oben", "unten", "vertikal" und "horizontal" verwendet werden, so beziehen sie sich ebenfalls auf die Anordnung des Grundkörpers: Die Basis des Grundkörpers befindet sich unten und die Spitze oben. Eine vertikale Richtung verläuft von unten nach oben. Dementsprechend verläuft eine horizontale Richtung senkrecht zur Vertikalen.

Der Grundkörper kann eine Außenwand umfassen, die dem Mantel des Kegel- oder Pyramidenstumpfs entspricht. Die Außenwand kann massiv ausgebildet sein. Sie kann auch durchbrochen sein. Insbesondere kann die Außenwand so ausgebildet sein, dass sie eine oder mehrere Streben umfasst, die von der Basis des Grundkörpers zu der Spitze des Grundkörpers verlaufen. Das spiralförmige Element kann an diesen Streben angeordnet sein. Die Streben können dabei insbesondere mittels des spiralförmigen Elements miteinander verbunden sein. Eine solche Ausbildung des Grundkörpers ist besonders vorteilhaft, da die Innenwände der Nase bei Verwendung der Vorrichtung weitestgehend freiliegen, was das Tragen der Vorrichtung angenehm gestaltet.

Die Außenseite der Außenwand kann glatt sein. Die Außenseite kann auch Erhebungen und/oder Vertiefungen und/oder stufenförmige Elemente aufweisen.

Bei der Verwendung der Vorrichtung wird der Naseneinsatz in ein Nasenloch eingeführt und im Nasenvorhof und zumindest teilweise in der Nasenhöhle platziert. Beim Einatmen durch das Nasenloch wird die Atemluft, die durch das Innere des Grundkörpers und über das spiralförmige Element strömt, in eine Drehbewegung um die Hauptachse des Grundkörpers versetzt. Die einströmende Luftsäule beginnt also um die Hauptachse zu rotieren. Diese Bewegung hat eine Sogwirkung zur Folge, durch welche die Menge von Atemluft, die in die Nase einströmt, erhöht wird.

Mit anderen Worten sorgt die erfindungsgemäße Vorrichtung alleine durch ihre geometrische Ausgestaltung dafür, dass die bei einem Atemzug einströmende Menge an Luft erhöht wird. Damit kann beispielsweise durch die Verwendung der Vorrichtung beim Schlafen ein erhöhter Erholungseffekt erzielt werden. Weiterhin kann auch ein Schnarchen unterdrückt werden. Auch kann die Verwendung der Vorrichtung beispielsweise bei einer sportlichen Tätigkeit die Sauerstoffaufnahme erhöhen und somit zu einer Leistungsförderung führen.

Vorzugsweise ist das Durchgangsloch ebenfalls konusförmig ausgebildet. Durch die in diesem Fall auftretende Verjüngung des Durchmessers des Lochs kann eine weitere Verstärkung des oben beschriebenen Effekts erzielt werden.

Da ein Großteil des erfindungsgemäßen Naseneinsatzes bei Verwendung im Nasenvorhof angeordnet ist, ist er leicht zugänglich und kann einfach vom Benutzer eingesetzt bzw. entfernt werden.

Da der oben erwähnte Effekt aufgrund der speziellen geometrischen Ausbildung des Naseneinsatzes auftritt, ist es nicht notwendig, den Naseneinsatz für jeden Benutzer individuell anzupassen. Der Naseneinsatz kann daher in verschiedenen Einheitsgrößen bereitgestellt werden, beispielsweise für Kinder und Erwachsene. Dies erlaubt eine effiziente Massenfertigung.

Der Grundkörper kann einen thermoplastischen Kunststoff insbesondere auf Kautschukbasis umfassen. Insbesondere kann der Grundkörper aus einem solchen Kunststoff, insbesondere aus einem thermoplastischen Elastomer, bestehen. Dies hat den Vorteil, dass die Vorrichtung für den Benutzer gut verträglich ist. Weiterhin sind solche Kunststoffe flexibel, so dass sich die äußere Form des Naseneinsatzes an den Nasenvorhof und/oder die Nasenhöhle anpassen kann. Auch erlaubt die Verwendung eines solchen Kunststoffs eine einfache Reinigung der Vorrichtung nach seiner Verwendung.

Der Kunststoff kann insbesondere frei von Weichmachern und/oder Polyphthalamiden (PPA) sein. Dies ist vorteilhaft, da der Naseneinsatz in direktem Kontakt mit der Nasenschleimhaut stehen kann. Durch den Verzicht auf Weichmacher kann verhindert werden, dass allergische Reaktionen auftreten. Auch ist bekannt, dass Weichmacher insbesondere für Kinder gesundheitsschädigend sein können.

Der Naseneinsatz kann beispielsweise durch ein Spritzgussverfahren hergestellt werden. Der Naseneinsatz kenn einteilig oder mehrteilig ausgebildet sein.

Der Grundkörper kann eine Höhe von 10 bis 50 mm aufweisen. Der Durchmesser bzw. die maximale Ausdehnung des Grundkörpers an seiner Basis in radialer Richtung kann 0,2 bis 25 mm betragen. Der Durchmesser bzw. die maximale Ausdehnung des Grundkörpers an seiner Spitze in radialer Richtung kann 0,2 bis 20 mm betragen. Die Wandstärke des Grundkörpers kann 0,1 bis 4 mm betragen. Die Wandstärke kann konstant sein, sie kann aber auch entlang der Höhe des Grundkörpers variieren.

Das spiralförmige Element kann eine Ganghöhe von 0,1 bis 5 mm besitzen. Die Ganghöhe bezeichnet dabei die Ganghöhe der oben erwähnten Schraublinie in Richtung der Hauptachse. Die Windungszahl der Spirale kann zwischen 1 und 20 betragen. Durch eine solche Ausgestaltung des spiralförmigen Elements kann eine wirbelförmige Drehbewegung der Luft besonders effektiv erzeugt werden.

Das spiralförmige Element kann äußere Abmessungen von 0,1 bis 5 mm aufweisen. Die Querschnittsform des spiralförmigen Elements kann polygon- oder kreis- bzw. ellipsenförmig sein.

Das spiralförmige Element kann weiterhin lamellenförmige Strukturen aufweisen. Alternativ oder zusätzlich können an der Innenseite des Grundkörpers lamellenförmige Strukturen angeordnet sein. Bei den lamellenförmigen Strukturen kann es sich um ein Bündel von Lamellen handeln. Zwischen diesen Lamellen können sich in der Atemluft enthaltener Feinstaub, Schmutz oder ähnliche Partikel ablagern. Dieser Effekt wird durch die wirbelförmige Drehbewegung der Luft unterstützt, aufgrund derer die einströmende Luft in Richtung der Innenwand gelenkt wird. Mit anderen Worten kann durch die Lamellen die einströmende Luft gefiltert werden. Die Verwendung eines der oben genannten Kunststoffe kann die Rückhaltefähigkeit der Lamellen zusätzlich erhöhen, da Kleinstpartikel gut an diesen Kunststoffen adhärieren.

Die Lamellen können sich entlang einer Schraublinie von der Basis des Grundkörpers zu seiner Spitze erstrecken. Die Lamellen können eine Höhe und/oder Dicke und/oder einen Abstand zueinander zwischen 0,1 und 4 mm besitzen. Durch eine solche Ausbildung der Lamellen kann eine besonders effektive Filterung der Luft erreicht werden.

Die Lamellen können vertikal oder horizontal angeordnet sein.

Weiterhin können die Lamellen so ausgebildet sein, dass Reservoirs für Stoffe zur Inhalation gebildet werden. Stoffe zur Inhalation können beispielsweise Stoffe zur Kurz- und Langzeitbehandlung von Atemwegserkrankungen, etwa von Bronchitis, Grippe- bzw. grippeähnlichen Symptomen und/oder Allergien sein. Die Stoffe können auch Stoffe zur Befreiung der Atemwege sein. Es ist auch möglich, dass es sich bei den Stoffen um Duftstoffe handelt.

Die Lamellen können ein dreieckförmiges Querschnittsprofil aufweisen. Hierbei liegt das Querschnittsprofil in einer Ebene, die normal zu der Schraublinie ist. Das Querschnittsprofil der Lamellen kann auch rechteckig sein. Es ist auch möglich, dass die Lamellen ein wellenförmiges oder gezacktes Querschnittsprofil aufweisen.

Weiterhin kann das Höhenprofil der Lamellen in Richtung der Schraublinie variieren. Beispielsweise kann das Höhenprofil der Lamellen von der Basis zu der Spitze des Grundkörpers entlang der Schraublinie zick-zack-förmig oder wellenförmig verlaufen.

Insbesondere können durch eine wellenförmige oder gezackte Ausbildung des Querschnitts- und/oder des Höhenprofils der Lamellen Vertiefungen entstehen, die als Reservoirs für die oben erwähnten Stoffe zur Inhalation dienen können.

Die Spitze des Grundkörpers kann dazu ausgebildet sein, in einen Nasengang eingeführt zu werden. Beispielsweise kann die Spitze des Grundkörpers aufgrund ihres Materials und ihrer Abmessungen eine ausreichende Flexibilität besitzen, um trichterförmig in einen Nasengang eingeführt zu werden. Die Atemluft kann so direkt durch den Naseneinsatz in den Nasengang strömen. Damit kann eine besonders effektive Leitung der Atemluft zum Nasenrachen erreicht werden.

Die Vorrichtung kann einen ersten Naseneinsatz und einen zweiten Naseneinsatz umfassen. Dabei kann der erste Naseneinsatz dazu vorgesehen sein, in ein erstes Nasenloch des Benutzers eingeführt werden. Der zweite Naseneinsatz kann dazu vorgesehen sein, in das zweite Nasenloch des Benutzers eingeführt werden. Dadurch kann der oben beschriebene vorteilhafte Effekt für beide Nasenlöcher und damit für beide Abschnitte der Nasenhöhle erzielt werden.

Die Vorrichtung kann weiterhin ein Verbindungselement umfassen, welches den ersten Naseneinsatz und den zweiten Naseneinsatz an der Basis ihrer jeweiligen Grundkörper strukturell miteinander verbindet. Insbesondere kann das Verbindungselement die jeweils der Nasenscheidewand zugewandten Seiten der Naseneinsätze miteinander verbinden. Das Verbindungselement kann beispielsweise brücken- oder stegartig ausgebildet sein. Das Verbindungselement kann fest oder lösbar mit den jeweiligen Naseneinsätzen verbunden sein.

Durch das Verbindungselement kann die Handhabung der Vorrichtung bei der Verwendung weiter verbessert werden. So kann beispielsweise sichergestellt werden, dass der Benutzer die Naseneinsätze korrekt in die Nasenlöcher einführt, da die Naseneinsätze durch das Verbindungselement die richtige Orientierung zueinander besitzen. Weiterhin kann das Verbindungselement die Naseneinsätze in den Nasenlöchern stabilisieren. Auch kann das Verbindungselement das Entfernen der Vorrichtung aus der Nase nach der Verwendung erleichtern. Weiterhin kann durch das Verbindungselement verhindert werden, dass bei Nicht-Verwendung einer der beiden Naseneinsätze verloren geht. Auch kann dadurch die Aufbewahrung der Vorrichtung erleichtert werden.

Bei einer derartigen Vorrichtung kann bei dem ersten Naseneinsatz an und/oder in der Außenseite des Grundkörpers ein erster Magnet angeordnet sein, wobei der erste Magnet bei Verwendung der Vorrichtung der Nasenscheidewand zugewandt ist. Weiterhin kann bei dem zweiten Naseneinsatz an der Außenseite der Wand an und/oder in der Außenseite des Grundkörpers ein zweiter Magnet angeordnet sein, wobei der zweite Magnet bei Verwendung der Vorrichtung der Nasenscheidewand zugewandt ist. Die Pole des ersten Magneten und des zweiten Magneten können derart angeordnet sein, dass bei Verwendung der Vorrichtung zwischen dem ersten Magnet und dem zweiten Magnet eine anziehende Kraft herrscht.

Durch eine derartige Ausgestaltung der Vorrichtung kann erreicht werden, dass der erste und der zweite Naseneinsatz aufgrund der Kraft, die zwischen den Magneten herrscht, gewissermaßen an der Nasenscheidewand "festgeklemmt" werden. Damit wird eine einfache und sichere Fixierung der Naseneinsätze in der Nase des Benutzers sichergestellt.

Die Stärke der Magnete kann beispielsweise 50 bis 100 mT betragen. Eine solche Stärke ermöglicht eine sichere Fixierung der Naseneinsätze, ohne dass sich die Kraft unangenehm für den Benutzer auswirkt. Auch sind solche Magnete in der benötigten Größe, insbesondere mit äußeren Abmessungen im Bereich von 0,2 bis 10 mm, einfach verfügbar. Die Magnete können beispielsweise Ferritmagnete sein. Es kann sich auch um Kunststoffmagnete handeln. Die Magnete können insbesondere flexible Magnete sein.

Die Magnete können vorzugsweise in die Wand des Grundkörpers eingebettet sein. So können die Magnete beispielsweise bereits während der Herstellung des Grundkörpers in die Wand eingebracht werden. Dies kann beispielsweise während des Spritzgießens geschehen. Das Einbetten der Magnete hat den Vorteil, dass die Außenseite der Wand glatt bleibt. Dies ermöglicht ein angenehmes Tragen der Vorrichtung für den Benutzer.

Alternativ oder zusätzlich ist es auch möglich, die Magnete auf der Innen- oder auf der Außenseite der Wand zu befestigen. Insbesondere ist es möglich, dass an der Außenseite der Wand eine Vertiefung oder Tasche angeordnet ist, in welcher ein Magnet befestigt wird. Dies kann beispielsweise mittels eines geeigneten Klebers geschehen. Insbesondere kann es sich dabei um einen lösungsmittelarmen Kleber, beispielsweise einen Dispersionskleber, handeln. Der Kleber kann beispielsweise ein Kleber auf Naturkautschukbasis sein. Solche Kleber haben den Vorteil, dass sie umweltfreundlich und unschädlich für den menschlichen Organismus sind.

Die Vorrichtung umfassend den ersten Naseneinsatz, den zweiten Naseneinsatz und das Verbindungselement kann einteilig ausgebildet sein. Sie kann auch mehrteilig ausgebildet sein.

Die Erfindung stellt weiterhin eine Verwendung einer Vorrichtung zur Verbesserung der Atmung, insbesondere zur nicht therapeutischen Leistungsförderung durch erhöhte Sauerstoffaufnahme, bereit, wobei mindestens ein Naseneinsatz in das Nasenloch eines Menschen eingeführt wird. Die Vorrichtung kann dabei eines oder mehrere der oben beschriebenen Merkmale aufweisen.

### Kurzbeschreibung der Zeichnungen

Weitere Merkmale und Vorteile der Erfindung werden nachfolgend anhand der beispielhaften Figuren erläutert. Dabei zeigt:
- Figur 1: schematisch eine Ausführungsform eines Naseneinsatzes in perspektivischer Ansicht;
- Figuren 2a bis 2e: schematisch ein spiralförmiges Element in Schnittdarstellung und perspektivischer Ansicht;
- Figur 3: schematisch einen Schnitt durch eine Ausführungsform eines Naseneinsatzes;
- Figur 4: schematisch einen Schnitt durch eine Ausführungsform der Vorrichtung, die zwei Naseneinsätze umfasst in Seitenansicht; und
- Figur 5: schematisch eine Ausführungsform Vorrichtung bei der zwei Naseneinsätze miteinander verbunden sind in Seitenansicht.

### Detaillierte Beschreibung

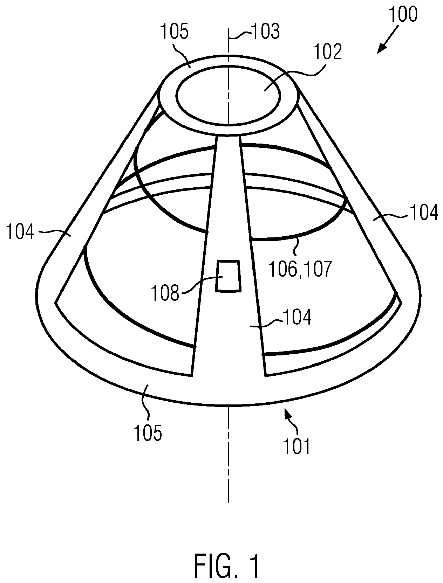

Figur 1 zeigt eine Ausführungsform eines Naseneinsatzes 100 für eine Vorrichtung 1 zur Verbesserung der Atmung eines Benutzers durch die Nase in perspektivischer Ansicht. Es ist zu erkennen, dass der Naseneinsatz 100 einen konusförmigen Grundkörper 101 umfasst. Weiterhin ist zu sehen, dass sich ein ebenfalls konusförmiges Durchgangsloch 102 entlang der Hauptachse 103, die in Figur 1 mittels der gestrichelten Linie angedeutet ist, durch den Grundkörper 101 erstreckt.

In der gezeigten Ausführungsform umfasst der Grundkörper 101 vier Streben 104, die von der Basis des Grundkörpers 101 zu seiner Spitze verlaufen. Die Streben 104 sind an ihrer Basis und an ihrer Spitze miteinander durch ringförmige Elemente 105 verbunden. Diese ringförmigen Elemente 105 können dem Grundkörper 101 eine erhöhte Stabilität verleihen, sie sind jedoch für die Funktionsweise der Vorrichtung 1 nicht notwendig und können auch weggelassen werden.

Es ist in Figur 1 weiterhin zu erkennen, dass im Inneren des Grundkörpers ein spiralförmiges Element 106 angeordnet ist. Das spiralförmige Element 106 verläuft in einer Schraublinie von der Basis des Grundkörpers 101 zu seiner Spitze. Die Streben 104 tragen das spiralförmige Element 106. In der gezeigten Ausführungsform umfasst das spiralförmige Element 106 drei Windungen. Es versteht sich jedoch, dass das spiralförmige Element 106 mehr oder weniger Windungen umfassen kann.

Der Grundkörper 101 und das spiralförmige Element 106 des in Figur 1 gezeigten Naseneinsatzes 100 bestehen aus einem Kunststoffmaterial, insbesondere einem Kunststoff auf Kautschukbasis. Dadurch ist der Naseneinsatz 100 für den Benutzer gut verträglich und angenehm zu tragen. Die Spitze des Grundkörpers 101 weist hierbei eine ausreichende Flexibilität auf, um sie in einen Nasengang eines Benutzers einzuführen.

In der in Figur 1 gezeigten Ausführungsform sind der Grundkörper 101 und das spiralförmige Element 106 einteilig ausgebildet.

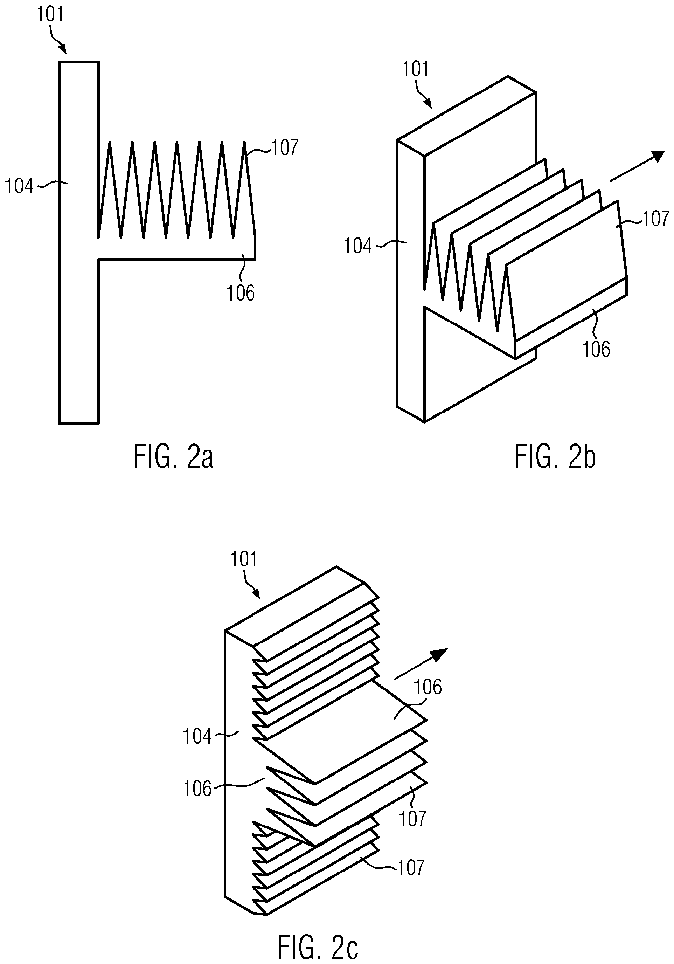

Das spiralförmige Element 106 umfasst weiterhin Lamellen 107 (in Figur 1 nicht gezeigt), die weiter unten mit Bezug auf die Figuren 2a bis 2c genauer beschrieben werden. Auch an der Innenseite des Grundkörpers 101, insbesondere an den Innenseiten der Streben 104, können Lamellen 107 angeordnet sein (in Figur 1 nicht gezeigt).

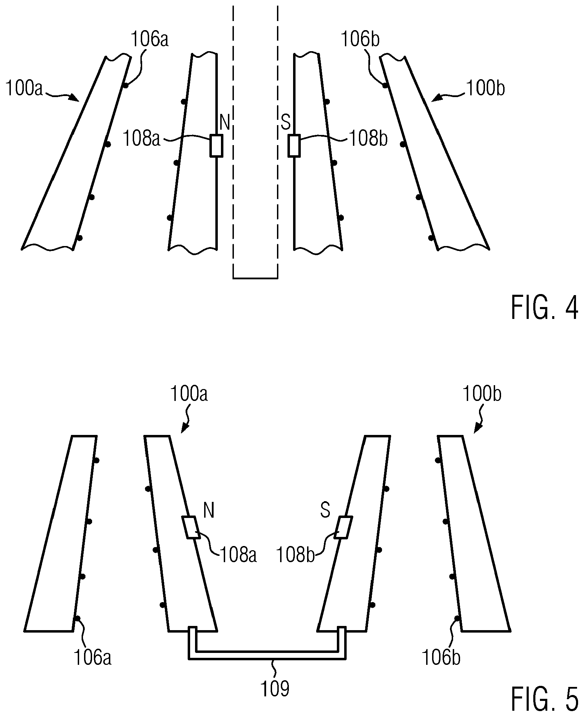

Figur 1 zeigt weiterhin, dass an einer Strebe 104 des Grundkörpers 101 ein Magnet 108 angeordnet ist. Die Funktion des Magneten 108 wird weiter unten mit Bezug auf die Figur 4 genauer erläutert.

Figur 2a zeigt schematisch eine Schnittdarstellung durch ein spiralförmiges Element 106. Die Zeichenebene in Figur 2a ist senkrecht zu der Schraublinie, entlang derer das spiralförmige Element 107 verläuft. In Figur 2a sind weiterhin der Grundkörper 101 sowie eine Strebe 104 zu sehen. Das spiralförmige Element 107 ist an der Strebe 104 im Inneren des Grundkörpers angeordnet. Figur 2a zeigt weiterhin, dass das spiralförmige Element 106 Lamellen 107 umfasst. Die Lamellen 107 sind in der gezeigten Ausführungsform aufrecht auf dem spiralförmigen Element 106 angeordnet und verlaufen in Richtung der Schraublinie.

Figur 2b zeigt die in Figur 2a illustrierten Elemente in einer perspektivischen Ansicht. In Figur 2b ist zu erkennen, dass die Lamellen 107 in Richtung der Schraublinie, die mittels des eingezeichneten Pfeils gekennzeichnet ist, verlaufen.

In der in Figur 2a und 2b gezeigten Ausführungsform sind die Lamellen 107 vertikal auf dem spiralförmigen Element 106 angeordnet sind. Alternativ oder zusätzlich können die Lamellen 107 vertikal unterhalb des spiralförmigen Elements 106 angeordnet sein.

In Figur 2c ist eine weitere Ausführungsform des spiralförmigen Elements in perspektivischer Ansicht gezeigt, bei der die Lamellen 107 horizontal angeordnet sind. Der eingezeichnete Pfeil bezeichnet wiederum die Richtung der Schraublinie. In Figur 2c ist weiterhin zu sehen, dass auch an der Innenseite des Grundkörpers 101, insbesondere an der Innenseite der Streben 104, Lamellen 107 angeordnet sein können. Diese verlaufen in der gezeigten Ausführungsform parallel zu den Lamellen 107, die auf dem spiralförmigen Element 106 angeordnet sind.

Der Abstand zwischen den in Figur 2a bis 2c gezeigten Lamellen 107 ist so gewählt, dass in der Atemluft vorhandene Partikel, wie Feinstaub oder Pollen zwischen den Lamellen 107 zurückgehalten werden können. So wird eine Filterung der Atemluft erreicht.

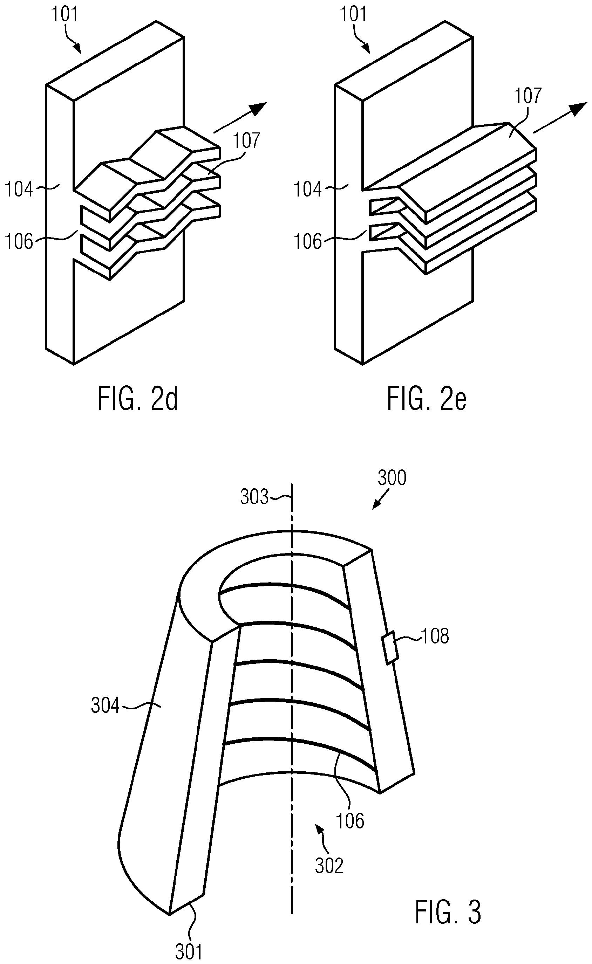

Bei den in den Figuren 2a bis 2c gezeigten Lamellen 107 nimmt die Dicke der Lamellen 107 von ihrer Basis zu ihrer Spitze hin ab. Mit anderen Worten haben die Lamellen 107 ein dreiecksförmiges Querschnittsprofil. Es sind aber auch andere Querschnittsprofile möglich. Es ist auch möglich, dass die Lamellen 107 eine konstante Dicke aufweisen, wie in den Figuren 2d und 2e gezeigt.

In Figur 2d ist eine weitere Ausführungsform des spiralförmigen Elements in perspektivischer Ansicht gezeigt, bei der die Lamellen 107 horizontal angeordnet sind. Der eingezeichnete Pfeil bezeichnet wiederum die Richtung der Schraublinie. Bei der in Figur 2d gezeigten Ausführungsform variiert das Höhenprofil der Lamellen 107 in Richtung der Schraublinie zick-zack-förmig. Alternativ könnte das Höhenprofil auch wellenförmig variieren.

Figur 2e zeigt eine weitere Ausführungsform des spiralförmigen Elements in perspektivischer Ansicht, bei der die Lamellen 107 horizontal angeordnet sind. Der eingezeichnete Pfeil bezeichnet wiederum die Richtung der Schraublinie. In dieser Ausführungsform ist das Querschnittsprofil der Lamellen 107 dachförmig ausgebildet.

Auch bei den in Figur 2d und 2e gezeigten Ausführungsformen können zusätzlich Lamellen an der Innenseite des Grundkörpers 101, insbesondere an der Innenseite der Streben 104, angeordnet sein (nicht gezeigt).

Weiterhin können zwischen den Lamellen 107 Stoffe zur Inhalation gelagert werden (nicht gezeigt). Mit anderen Worten können die Lamellen 107 als Reservoir für die Stoffe zur Inhalation dienen. Bei der in Figur 2a und 2b gezeigten Ausführungsform können die Stoffe zwischen den vertikal angeordneten Lamellen gelagert 107 werden. Bei den in Figur 2d und 2e gezeigten Ausführungsformen können die Stoffe in den Vertiefungen, die durch die Variation des Höhenprofils bzw. des Querschnitts gebildet werden, gelagert sein.

Figur 3 zeigt eine alternative Ausführungsform eines Naseneinsatzes 300 für eine Vorrichtung 1 zur Verbesserung der Atmung eines Benutzers durch die Nase in Schnittdarstellung. Es ist zu erkennen, dass der Naseneinsatz 300 einen konusförmigen Grundkörper 301 umfasst. Weiterhin ist zu sehen, dass sich ein ebenfalls konusförmiges Durchgangsloch 302 entlang der Hauptachse 303, die in Figur 3 mittels der gestrichelten Linie angedeutet ist, durch den Grundkörper 301 erstreckt.

Der in Figur 3 gezeigte Naseneinsatz 300 besteht aus einem Kunststoffmaterial, insbesondere einem Kunststoff auf Kautschukbasis. Dadurch ist der Naseneinsatz 300 für den Benutzer gut verträglich und angenehm zu tragen. Die Spitze des Grundkörpers 301 weist hierbei eine ausreichende Flexibilität auf, um sie in einen Nasengang eines Benutzers einzuführen.

Durch den Grundkörper 301 und das Durchgangsloch 302 wird die Wand 304 des Grundkörpers 301 gebildet. Im Inneren des Grundkörpers ist ein spiralförmiges Element 106 angeordnet, das entlang einer Schraublinie von der Basis des Grundkörpers 301 bis zu der Spitze des Grundkörpers 301 verläuft. In der gezeigten Ausführungsform umfasst das spiralförmige Element 106 fünf Windungen. Es versteht sich jedoch, dass das spiralförmige Element 106 mehr oder weniger Windungen umfassen kann.

Figur 3 zeigt weiterhin, dass an einer Außenseite des Grundkörpers 301 ein Magnet 108 angeordnet ist. Die Funktion des Magneten 108 wird weiter unten mit Bezug auf die Figur 4 genauer erläutert.

Der in Figur 3 gezeigte konusförmige Grundkörper 301 hat die Form eines geraden Kegelstumpfs. Es ist jedoch auch möglich, dass der Grundkörper 301 die Form eines schiefen Kegelstumpfes aufweist. Beispielsweise kann der Kegelstumpf zu der Seite geneigt sein, die bei Verwendung des Naseneinsatzes 300 der Nasenscheidewand zugeneigt ist. In diesem Fall ist es vorteilhaft, wenn der Magnet 108 an dieser Seite angeordnet ist. Auch andere geometrische Formen, die eine sich nach oben verjüngende Struktur aufweisen, sind mögliche Formen für den Grundkörper 301.

Das in Figur 3 gezeigte spiralförmige Element 106 kann wie das weiter oben beschriebene spiralförmige Element ausgebildet sein. Insbesondere kann es, wie in den Figuren 2a bis 2e gezeigt, Lamellen 107 umfassen.

Auch bei der in Figur 3 gezeigten Ausführungsform können Lamellen an der Innenseite der Wand 304 angeordnet sein. Dabei können die Lamellen ebenfalls entlang einer Schraublinie von der Basis der Grundkörpers 301 bis zu seiner Spitze verlaufen, entsprechend der in Figur 2c gezeigten Ausführungsform.

Figur 4 zeigt schematisch einen Ausschnitt einer Ausführungsform einer Vorrichtung 1 zur Verbesserung der Atmung in seitlicher Schnittansicht. Dabei illustriert Figur 4 eine Situation, bei der die Naseneinsätze 100a und 100b in die Nasenlöcher eines Benutzers eingeführt worden sind.

Die gezeigte Vorrichtung 1 umfasst zwei Naseneinsätze 100a und 100b, die dazu ausgebildet sind, in die zwei Nasenlöcher eines Benutzers eingeführt zu werden. Dabei können die Naseneinsätze 100a und 100b prinzipiell jeweils dem in Figur 1 gezeigten Naseneinsatz 100 entsprechen oder dem in Figur 3 gezeigten Naseneinsatz 300 entsprechen. Es sind die jeweiligen spiralförmigen Elemente 106a und 106b und die Magnete 108a und 108b zu erkennen. Die Naseneinsätze 100a und 100b unterscheiden sich bei der gezeigten Ausführungsform in der Anordnung der Magnete 108a und 108b. So ist der Nordpol des Magneten 108a nach außen gewandt, während der Magnet 108b so angeordnet ist, dass sein Südpol nach außen zeigt.

Der in Figur 4 gezeigte Ausschnitt der Naseneinsätze 100a und 100b zeigt die Umgebung der Magnete 108a und 108b. Weiterhin ist die Nasenscheidewand des Benutzers durch die gestrichelten Linien angedeutet. Es ist zu erkennen, dass die Außenwände der Naseneinsätze 100a und 100b aufgrund der magnetischen Anziehungskraft zwischen dem Nordpol des Magneten 108a und dem Südpol des Magneten 108b gegen die Nasenscheidewand gedrückt werden. Somit wird ein sicherer Halt der Naseneinsätze 100a und 100b in der Nase erreicht.

In alternativen Ausführungsformen können auch mehrere Magnete 108 in der Wand eines Naseneinsatzes angeordnet sein. So ist es beispielsweise möglich, dass sich eine Reihe von Magneten 108 von der Basis des Grundkörpers bis zu der Spitze des Grundkörpers erstreckt.

Figur 5 zeigt eine weitere Ausführungsform einer Vorrichtung 1 zur Verbesserung der Atmung in Seitenansicht. Diese Ausführungsform umfasst dieselben Elemente wie die in den Figuren 1 bis 4 gezeigten Ausführungsformen, die hier nicht nochmals näher beschrieben werden. Dabei können die Naseneinsätze 100a und 100b prinzipiell jeweils dem in Figur 1 gezeigten Naseneinsatz 100 entsprechen oder dem in Figur 3 gezeigten Naseneinsatz 300 entsprechen. In der in Figur 5 illustrierten Vorrichtung ist weiterhin ein Verbindungselement 109 vorgesehen, welches die Naseneinsätze 100a und 100b an ihrer jeweiligen Basis miteinander verbindet. Es ist zu erkennen, dass das Verbindungselement 109 die Seiten der Naseneinsätze 100a und 100b miteinander verbindet, an denen die Magnete 108a und 108b angeordnet sind.

Durch das Verbindungselement 109 kann zum einen sichergestellt werden, dass der Benutzer die Naseneinsätze 100a und 100b in der richtigen Orientierung in die Nasenlöcher einführt. Insbesondere sind aufgrund der Verbindung durch das Verbindungselement 109 die Magnete 108a und 108b einander automatisch zugewandt. Weiterhin kann das Verbindungselement 109 die in die Nasenlöcher eingeführten Naseneinsätze 100a und 100b bei der Verwendung der Vorrichtung 1 stabilisieren. Das Verbindungselement 109 kann auch dabei helfen, die Vorrichtung 1 nach der Verwendung wieder aus der Nase zu entfernen. So kann der Benutzer das Verbindungselement 109 dazu verwenden, die Naseneinsätze 100a und 100b aus den jeweiligen Nasenlöchern heraus zu ziehen. Weiterhin kann das Verbindungselement 109 verhindern, dass in der Situation, in der die Vorrichtung 1 sich nicht in der Nase des Benutzers befindet, einer der Naseneinsätze 100a und 100b verloren geht. Auch die Aufbewahrung der Vorrichtung 1 wird hierdurch erleichtert.

Das Verbindungselement 109 ist bei der in der Figur 5 gezeigten Ausführungsform fest mit den Naseneinsätzen 100a und 100b verbunden. Es ist aber auch möglich, dass das Verbindungselement 109 ein eigenständiges Element ist, welches lösbar mit den Naseneinsätzen 100a und 100b verbunden ist. Dies kann beispielsweise über eine geeignete Steckverbindung erzielt werden, welche das Verbindungselement 109 form- und/oder kraftschlüssig mit den Naseneinsätzen verbindet. So können die Enden des Verbindungselements 109 zapfenförmig ausgebildet sein und dazu vorgesehen sein, jeweils in eine entsprechende Öffnung oder Öse in den Naseneinsätzen 100a und 100b gesteckt zu werden. Solche Verbindungsmöglichkeiten sind beispielsweise von Sicherheitsfäden zur Befestigung von Etiketten bekannt.

Bei der Verwendung der Vorrichtung 1 führt der Benutzer zunächst den Naseneinsatz 100a in eines seiner Nasenlöcher und den Naseneinsatz 100b in das andere Nasenloch ein. Dabei kann der Benutzer die Naseneinsätze so einführen, dass die Spitze des Naseneinsatzes 100a und/oder die Spitze des Naseneinsatzes 100b jeweils in einen jeweiligen Nasengang eingeführt wird.

Durch die Anziehungskraft zwischen den Magneten 108a und 108b wird die Vorrichtung an der Nasenscheidewand des Benutzers festgeklemmt. Der Benutzer kann dann durch seine Nasenlöcher einatmen, wobei die Atemluft aufgrund der spiralförmigen Elemente 106a und 106b in eine Drehbewegung versetzt wird und so in der oben beschriebenen Art und Weise ein Sogeffekt und eine erhöhte Luftzufuhr durch die Nase erreicht wird.

Die Vorrichtung 1 kann nach Belieben des Benutzers in der Nase verbleiben. So ist es möglich, die Vorrichtung 1 während des Schlafes zu tragen.

Zum Entfernen der Vorrichtung 1 greift der Benutzer die Naseneinsätze 100a und 100b und zieht sie aus den jeweiligen Nasenlöchern heraus. Dies kann auch unter Verwendung des Verbindungselements 109 geschehen.

Nach der Verwendung kann die Vorrichtung 1 einfach mittels Wasser und/oder geeinigter Reinigungsmittel und/oder mittels Ultraschallreinigung gereinigt werden.

Es versteht sich, dass in den zuvor beschriebenen Ausführungsbeispielen genannte Merkmale nicht auf diese speziellen Kombinationen beschränkt und auch in beliebigen anderen Kombinationen möglich sind. Weiterhin versteht es sich, dass in den Figuren gezeigte Geometrien nur beispielhaft sind und auch in beliebigen anderen Ausgestaltungen möglich sind.

## Patentansprüche

1. Vorrichtung (1) zur Verbesserung der Atmung, umfassend:
mindestens einen Naseneinsatz (100), der dazu vorgesehen ist, in ein Nasenloch eines Benutzers eingeführt zu werden,
wobei der mindestens eine Naseneinsatz (100) umfasst:
einen konusförmigen Grundkörper (101);
ein Durchgangsloch (102), das sich entlang der Hauptachse (103) des Grundkörpers (101) erstreckt; und
ein spiralförmiges Element (106), das innerhalb des Grundkörpers (101) angeordnet ist und sich von der Basis des Grundkörpers (101) bis zur Spitze des Grundkörpers (101) erstreckt.

2. Vorrichtung gemäß Anspruch 1, wobei das spiralförmige Element (106) eine Ganghöhe von 0,1 bis 5 mm besitzt und/oder wobei das spiralförmige Element (106) eine Windungszahl von 1 bis 20 aufweist.

3. Vorrichtung gemäß einem der vorhergehenden Ansprüche, wobei Grundkörper eine oder mehrere Streben (104) umfasst, an denen das spiralförmige Element (106) angeordnet ist.

4. Vorrichtung gemäß einem der vorhergehenden Ansprüche, wobei das spiralförmige Element (106) und/oder die Innenseite des Grundkörpers (101) lamellenförmige Strukturen (107) umfasst.

5. Vorrichtung gemäß Anspruch 4, wobei sich die lamellenförmigen Strukturen (107) entlang einer Schraublinie von der Basis zu der Spitze des Grundkörpers erstrecken.

6. Vorrichtung gemäß Anspruch 4 oder 5, wobei die lamellenförmigen Strukturen (107) eine Höhe und/oder Dicke und/oder einen Abstand zueinander zwischen 0,1 und 4 mm besitzen.

7. Vorrichtung gemäß einem der Ansprüche 4 bis 6, wobei die lamellenförmigen Strukturen (107) so ausgebildet sind, dass Reservoirs für Stoffe zur Inhalation gebildet werden.

8. Vorrichtung gemäß einem der vorhergehenden Ansprüche, wobei die Vorrichtung (1) einen ersten Naseneinsatz (100a) und einen zweiten Naseneinsatz (100b) umfasst.

9. Vorrichtung gemäß Anspruch 8,
wobei bei dem ersten Naseneinsatz (100a) an und/oder in der Außenseite (105) des Grundkörpers (101), ein erster Magnet (108a) angeordnet ist, wobei der erste Magnet (108a) bei Verwendung der Vorrichtung (1) der Nasenscheidewand zugewandt ist,
wobei bei dem zweiten Naseneinsatz (100b) an und/oder in der Außenseite (105) des Grundkörpers (104), ein zweiter Magnet (108b) angeordnet ist, wobei der zweite Magnet (108b) bei Verwendung der Vorrichtung (1) der Nasenscheidewand zugewandt ist, und
wobei die Pole des ersten Magneten (108a) und des zweiten Magneten (108b) derart angeordnet sind, dass bei Verwendung der Vorrichtung (1) zwischen dem ersten Magnet (108a) und dem zweiten Magnet (108b) eine anziehende Kraft herrscht.

10. Vorrichtung gemäß Anspruch 8 oder Anspruch 9, weiterhin umfassend ein Verbindungselement (109), welches den ersten Naseneinsatz (100a) und den zweiten Naseneinsatz (100b) an der Basis ihrer jeweiligen Grundkörper (101) miteinander verbindet.

11. Vorrichtung gemäß einem der vorhergehenden Ansprüche, wobei der Grundkörper (101) des mindestens einen Naseneinsatzes (100) eine Höhe von 10 bis 50 mm besitzt und/oder wobei eine Wand des mindestens einen Naseneinsatzes (100) eine Stärke von 0,1 bis 4 mm aufweist.

12. Vorrichtung gemäß einem der vorhergehenden Ansprüche, wobei der Durchmesser des Grundkörpers (101) des mindestens einen Naseneinsatzes (100) an seiner Basis 0,2 bis 25 mm beträgt und/oder der Durchmesser des Grundkörpers (101) des mindestens einen Naseneinsatzes (100) an seiner an seiner Spitze 0,2 bis 20 mm beträgt.

13. Vorrichtung gemäß einem der vorhergehenden Ansprüche, wobei der Grundkörper (101) einen thermoplastischen Kunststoff insbesondere auf Kautschukbasis umfasst.

14. Verwendung der Vorrichtung gemäß einem der Ansprüche 1 bis 13 zur Verbesserung der Atmung, wobei der mindestens eine Naseneinsatz (100) in das Nasenloch eines Benutzers eingeführt wird.
